# EUROPEAN PATENT SPECIFICATION

(11) **EP 1 176 154 B1**
(45) Date of publication and mention of the grant of the patent: **29.06.2005**
(21) Application number: 01117259.0
(22) Date of filing: 17.07.2001
(51) Int. Cl.: C08B 37/16, A61K 31/724, A61K 38/05, A61K 31/4164

(54) **Carnosine derivatives, a process for the preparation thereof and pharmaceutical compositions containing them**
Carnosinderivate, Verfahren zur Herstellung und diese enthaltende pharmazeutische Zusammensetzungen
Dérivés de carnosine, procédé de préparation et compositions pharmaceutiques les contenant

(30) Priority: 25.07.2000 IT MI001696
(43) Date of publication of application: 30.01.2002
(73) Proprietor: Universita' Degli Studi Di Catania, 95100 Catania (IT)
(72) Inventor: Rizzarelli, Enrico, V.le A. Dorio 8, 95125 Catania (IT); Vecchio, Graziella, V.le A. Dorio 8, 95125 Catania (IT); La Mendola, Diego, V.le A. Dorio 8, 95125 Catania (IT)
(74) Representative: Minoja, Fabrizio, Dr.

(56) References cited:
- F. DJEDAINI-PILARD ET AL.: "Synthesis of a new molecular carrier: N-(Leu-enkephalin)yl 6 amido-6-deoxy-cyclomaltoheptaose" TETRAHEDRON LETTERS, vol. 34, no. 15, 1993, pages 2457-2460, XP002184077
- K. NAGAI ET AL.: "Nucleobase-functionalized beta-cyclodextrins. Preparation and spectral properties" JOURNAL OF ORGANIC CHEMISTRY, vol. 49, 1984, pages 1022-1027, XP002184078

## Description

The present invention relates to carnosine (β-alanylhistidine) derivatives of formula (I) 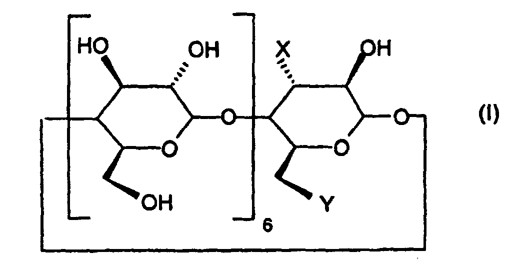 wherein X and Y, which are different from each other, are a chain deriving from carnosine or an OH group.

Compounds of formula (I) are obtained by functionalizing the 3- or 6-positions of β-cyclodextrin with carnosine. In particular, the invention relates to 3^{A}(R)-deoxy-3^{A}(R)-(β-alanylhistidine)-2^{A}(S)-β-cyclodextrin, 6-deoxy-6-(β-alanylhistidine)-β-cyclodextrin and the 6-deoxy-6-(β-alanylhistidylamino)-β-cyclodextrin, of formulae (Ia), (Ib), (Ic), respectively:

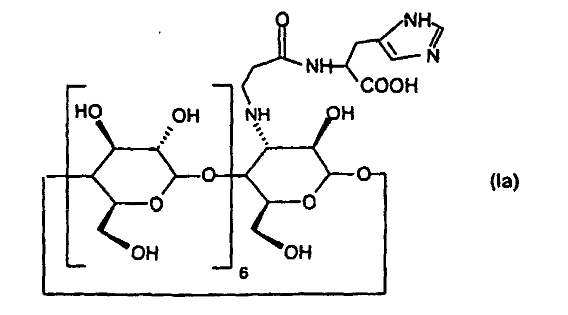

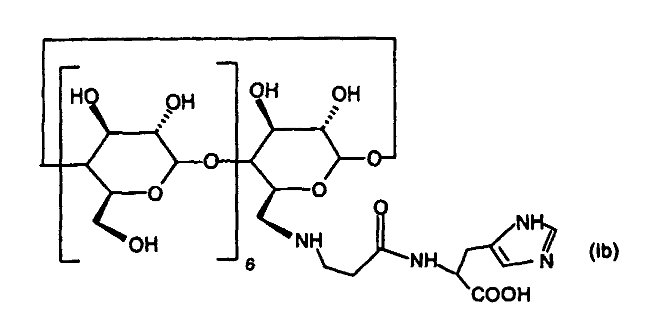

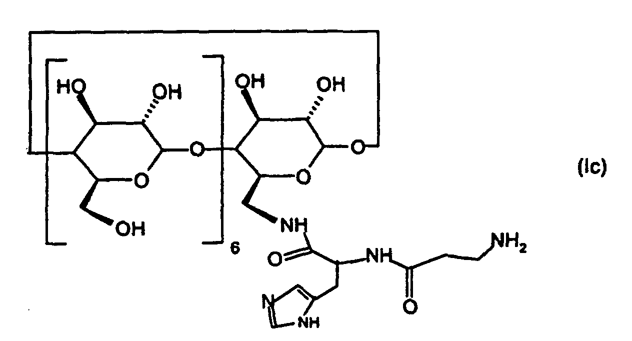

The invention further relates to the processes for the preparation of the compounds of formula (I). Finally, the invention also relates to pharmaceutical compositions with antioxidant (radical scavenger) activity and, in particular, anticataract activity.

Life in oxygen containing environment requires a protection system based on molecules with antioxidant activity, to protect molecules important for life, such as proteins, lipids, nucleic acids, and like. The higher level of oxidative metabolism in vertebrates is characteristic of muscles and brain. As a consequence, in these tissues a high concentration of small molecules with antioxidant activity, such as glutathione and other linear or cyclic dipeptides, has been found, mainly carnosine which is an excellent OH and singlet oxygen radical scavenger (Babizhayev M.A., et al, Biochem. J. 967 (1988)241). In relation with this activity, the possibility to use carnosine to prevent and partially treat cataract has been suggested (Boldyrev A.A., et al, Biochem. Int. 15 (1987) 1105; Babizhayev M.A., Biochim. Biophys Acta 1004 (1989)363). The main problem with the use of carnosine is related with its degradation by carnosinase or its elimination, therefore a natural derivative, N-acetyl-carnosine, has been used as a potential medicament against cataract (PCT/EP 94/03340). N-acetyl carnosine is rather resistant to carnosinase, and thus it is capable of maintaining comparatively high concentrations of active ingredient. N-acetylcarnosine has however some drawbacks from the practical standpoint.

It has now been found that is possible to obtain carnosine derivatives having high radical scavenging characteristics as well as different capability of being metabolized by the biological systems by conjugating carnosine with cyclodextrin, in particular with β-cyclodextrin. These cyclic oligosaccharides have been used as carriers also in the case of other peptides (see e.g. H. Parrot-Lopez et al, Tetrahedron Lett. 31 (1990), 1999; F. Djedaini-Pilard et al, ibid., 34 (1993), 2457; N. Schaschke et al, J. Am. Chem. Soc. 120 (1998), 7030).

In view of the different nature of the substrates and enzymatic systems involved, it should be considered surprising that the carnosine derivatives of the present invention have, compared with N-acetylcarnosine, the advantages which will be illustrated in the following.

According to the invention, the compounds of formula (I) are obtained by reacting suitably functionalized β-cyclodextrin with alanylhistidine optionally protected at the amino or carboxylic groups. The functionalized β-cyclodextrin can be, for example, 6-deoxy-6-iodo-β-cyclodextrin, epoxy-3A,2B-anhydrocyclodextrin or 6-amino-6-deoxy-β-cyclodextrin, whereas alanylhistidine can be used, depending on the cases, as such or in the form of ester (e.g. methyl) or as N-benzyloxycarbonyl derivative.

The following examples further illustrate the invention.

Commercial reagents were used, unless otherwise specified, which were treated, when necessary, as follows: β-cyclodextrin (CD) (Sigma) was dried under vacuum (10⁻² mm Hg) for about 24h at 80°C, using a P₂O₅ trap. Dry dimethylformamide was purchased from Aldrich; β-carnosine methyl ester (AlaHisOCH₃) was prepared from β-carnosine (AlaHis) (Sigma) with HCl in MeOH at 0°C, using acetyl chloride as HCl source. The thin layer chromatography (TLC) was carried out on silica gel (60F-254, 0.25 mm, Merck) and the products, not visible under UV light, were revealed using a 5% anysaldehyde solution in ethanol (containing 5% H₂SO₄) and Pauli's reagent for carnosine derivatives.

¹H-NMR spectra were recorded on a Bruker AC-400 MHz spectrometer at 400 MHz. ¹³C-NMR spectra were recorded on Bruker spectrometer at 100 MHz. All the solutions were prepared in D₂O, and the HDO frequency was taken as the reference one, in order to avoid any interferences of the conventional reference standards with the cyclodextrin cavity.

### Example 1

### 3^{A}(R)-Deoxy-3^{A}(R)-(β-alanylhistidine)-2^{A}(S)-β-cyclodextrin (CDAlaHis (3)). (Formula (Ia))

The synthesis was carried out starting from 3-O-(p-tosyl)-β-cyclodextrin through cyclodextrin epoxide 3A,2B-anhydrocyclodextrin (Breslow R., et al, J. Am.Chem. Soc., 108, 1069 (1986)).

The solution containing the epoxide (0.1 g) was added with AlaHis (5 times the stoichiometric amount). The reaction was carried out at 60°C, under N₂ stream, for 12 h. Water was evaporated and the resulting solid was purified using a chromatographic column with Rp 8, which was eluted first with water and then with a water/MeOH linear gradient (O→20%). The resulting product was further purified on CM-Sephadex C-25, using as water eluent. The resulting solid was collected by dissolution in the minimum amount of water and precipitation with acetone. Yield: 30%, Rf= 0.56, eluent PrOH/H₂O/NH₃ 5:3:1. FAB MS 1343 (M+1); Anal. Calcd. C₅₁H₈₂N₄O₃₇.4H₂O C 43.3, H 6.4, N, 3.9, found C 41.2, H 6.2, N 3.7).

¹H NMR (D₂O)δ(ppm) 8.0 (s, 1H, H-2, imidazole ring), 7.08 (s, 1 H, H-5, imidazole), 5.2-4.9 (m, 7H, H-1 of CD), 4.54 (m, 1H, CH, histidine chain), 4.29 (m, 1H, H-5A), 4.25 (t, 1H, H-4A), 4.1-3.75 (m, 27 H, H-3, -5, - 6, -2A), 3.75-3.5 (m, 13H, H-2, -4), 3.3 (dd, 1H, H-3A), 3.29 (m, 1H, proton of His CH₂), 3.2 (m, 2H, CH₂ of the Ala chain), 3.05 (dd, 1H, other proton of His CH₂), 2.67 (m, 2H, CH₂ of Ala adjacent to the NH group). ¹³C NMR (D₂O), δ (ppm) 179.9 (COO-), 175.2 (C(O)NH), 137.3 (C-2, imidazole ring), 134.4 (C-5, imidazole), 120.3 (C-4, imidazole), 105.8 (C-1A), 105-103 (other C-1), 83-81 (C-4), 76.7 (C-4A) 76-73.6 (C-2, C-3, C-5), 62.9 (C-6) 62.4 (C-6A), 61.0 (C-3A), 57.4 (CH, His residue), 45.1 (CH₂, Ala residue), 43.6 (CH₂, Ala residue), 31.0 (CH₂, His residue).

### Example 2

### 6-Deoxy-6-(β-alanylhistidine)-β-cyclodextrin (CDAlaHis (6)) (formula (Ib))

The cyclodextrin functionalized at the 6- position with carnosine was prepared starting from 6-O-(p-tosyl)-β-cyclodextrin, using 6-deoxy-6-iodo-β-cyclodextrin as an intermediate (CDI), as described above (Bonomo R.P. et al, Inorg. Chem., 30. 2708 (1991)).

Previously dried CDI (0.1 g) was dissolved in dry DMF (1 ml), purged with nitrogen, then β-carnosine (AlaHisOCH₃) was added (in 1:5 molar ratio to CDI). The reaction was carried out at 70°C, under stirring and nitrogen atmosphere, then after 12 hrs DMF was evaporated off under vacuum at 40°C. The resulting solid was loaded on a cation exchange column CM-Sephadex C-25 (in the NH₄⁺ form), using first water as eluent, then a NH₄CO₃ (0-0.1 M) linear gradient. The collected fractions were checked by thin layer chromatography (TLC), and those containing the product were concentrated under vacuum at 40°C. The resulting CDAlaHis methyl ester was hydrolyzed with 1% NaOH in methanol/water for about 1h. As the starting product is sparingly soluble in this solvent, the reaction was carried out in heterogeneous phase. On the other hand, the final product is highly soluble and, after completion of the reaction, the solution is quite clear. The solvent was evaporated off, the resulting solid was again purified by a CM-Sephadex C-25 column using water as eluent. Yield: 20%, Rf = 0.56, eluent PrOH/H₂O/NH₃ 5:3:1. FAB MS 1343 (M+1); Anal. Calcd. C₅₁H₈₂N₄O₃₇.8H₂O C 41.2, H 6.6, N, 3.8, found C 40.9, H 6.4, N 3.7).

¹H NMR (D₂O) δ (ppm) 7.84 (s, 1H, H-2, imidazole ring), 6.95 (s, 1H, H-5, imidazole), 5.1-5.0 (m, 7H, H-1, CD), 4.40 (m, 1H, CH, histidine chain), 4.02 (m, 1H, H-5A), 4.1-3.7 (m, 26H, H-3, -5, -6), 3.7-3.4 (m, 14 H, H-2, -4), 3.47 (m, 1H, H-6'A), 3.22-3.05 (m, 4H, H-6A, H of His CH₂, Ala CH₂), 2.95 (dd, 1H, other proton of His CH₂), 2.60 (m, 2 H other Ala CH₂).

¹³C NMR (D₂O), δ (ppm) 179.9 (COO-), 174.5 (C(O)NH), 137.7 (C-2, imidazole ring), 135.1 (C-5, imidazole), 119.9 (C-4, imidazole), 104.5-103.8 (C-1), 85.8 (C4-A), 83.4-83 (other C-4) 75.7-74.5 (C-2, C-3, C-5), 70.6 (C-5A), 63.2-62.8 (C-6), 57.5 (CH, His residue), 50.8 (C-6A), 46.9 (CH₂, Ala residue), 34.3 (CH₂, Ala residue), 31.2 (CH₂, His residue).

### Example 3

### Preparation of 6-deoxy-6-(β-alanylhistidylamino)-β-cyclodextrin (CDNHAlaHis) (formula (Ic))

The cyclodextrin functionalized at the 6- position with carnosine linked through the carboxyl was prepared by a condensation reaction starting from 6-amino-6-deoxy-β-cyclodextrin (CDNH₂), in its turn prepared starting from CDN3 (H.Parrot-Lopezet et al, Tetrahedron Lett., 1990, 31, 1999). A solution of CDN3 (0.5 g) in dry DMF (5 ml) at 45°C was added with triphenylphosphine (0.34 g). After 1h, ammonia (1 ml, 25% solution) was added and the system was kept at room temperature for 12h. After completion of the reaction, the solvent was evaporated off and the resulting solid was washed with acetone. The product was recovered by chromatography on a Sephadex C-25 column (35 x 900 mm) (NH₄⁺ form), using a solution of NH₄HCO₃ as eluent with linear gradient 0 - 0.1M (2 L). The fractions containing the product were collected, the solvent was evaporated, and the residual solid was repeatedly taken up with water, which was evaporated from time to time to remove NH₄CO₃.

Yield 25 %. Rf=0.63, eluent PrOH/H₂O/AcOEt/NH₃ 5:3:2:1. ¹H NMR (D₂O, 500 MHz) δ (ppm): 4.96 (m, 7H, H-1), 3.87-3.74 (m, 26H, H-3,5,6), 3.55-3.45 (m, 13H, H-2,4), 3.36 (t, 1H, H-4A), 3.00 (dd, 1H, H-6'A), 2.78 (dd, 1H, H-6A).

The β-carnosine amino-protected with the Boc group (AlaHisBoc) (0.035 g), dissolved in DMF (1 ml), was added with an equimolecular amount of 1-hydroxybenzotriazole (HOBT), as activating agent, and [(O-benzotriazole-1-yl)N,N,N',N'-bis(tetramethylene)uronium-hexafluorophosphate] (BBC), as condensing agent. Said solution was kept under stirring for 10 min, then added with CDNH₂ (0.1 g). The reaction was carried out at room temperature, under stirring and nitrogen; after 2 hrs DMF was evaporated off under vacuum at 40°C. The resulting solid was loaded on a cation exchange column CM-Sephadex C-25 (in NH₄⁺ form), using as eluent first water, then a linear gradient of NH₄CO₃ (0-0.1M). The collected fractions were controlled by thin layer chromatography (TLC), and those containing the product were concentrated under vacuum at 40°C. The product was dissolved in CF₃COOH to remove the Boc group at room temperature for 2h. The solvent was evaporated off, the resulting solid was purified again by a CM-Sephadex C-25 column using water as eluent. Yield: 25%, Rf= 0.32, eluent PrOH/H₂O/NH₃ 5:3:1. FAB MS 1342 (M+1); Anal. Calcd. C₅₁H₈₃O₃₆.6H₂₀ C 42.2, H 5.7, N, 4.8, found C 41.5, H 5.5, N 4.6).

¹H NMR (D₂O) δ (ppm) 7.73 (s, 1H, H-2, imidazole ring), 6.97 (s, 1H, H-5, imidazole), 5.15-4.96 (m, 7H, H-1 of the CD), 4.64 (m, 1H, CH, His chain), 4.00-3.76 (m, 26 H, H-3,-5,-6), 3.76-3.57 (m, 14 H, H-2,-4), 3.48 (m, 1H, H-6A), 3.22 (m, 1H 4H), 3.10-2.97 (m, 4H, His CH₂ and Ala CH₂), 2.60 (m, 2H, other Ala CH₂).

## Claims

1. Compounds of formula (I): 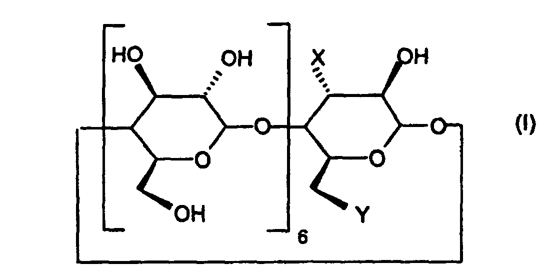 wherein X and Y, which are different from each other, are a chain deriving from carnosine (β-alanylhistidine), or a OH group.

2. As compound as claimed in claim 1, 3^{A}(R)-deoxy-3^{A}(R)-(β-alanylhistidine)-2^{A}(S)-β-cyclodextrin, of formula (Ia)

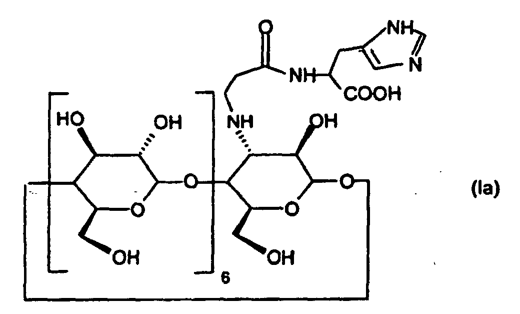

3. As compound as claimed in claim 1, 6-deoxy-6-(β-alanylhistidine)-β-cyclodextrin, of formula (Ib)

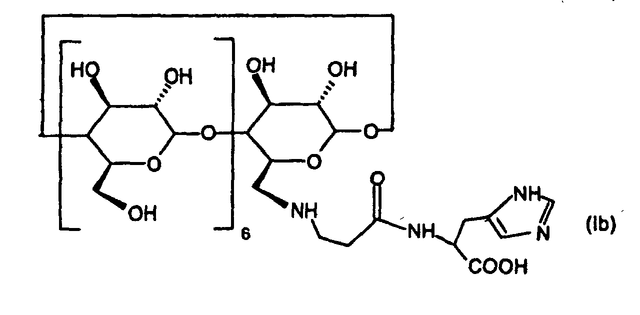

4. As compound as claimed in claim 1, 6-deoxy-6-(β-alanylhistidylamino)-β-cyclodextrin, of formula (Ic)

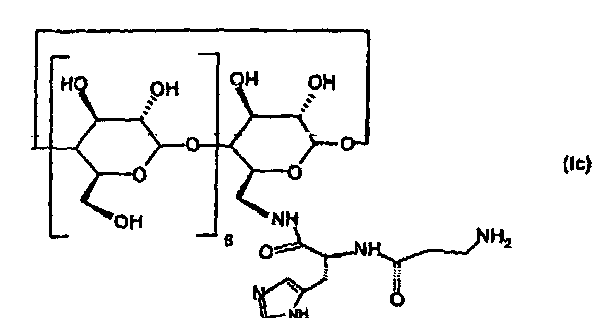

5. A process for the preparation of the compounds as claimed in claim 1, **characterized in that** a functionalized β-cyclodextrin is reacted with alanylhistidine optionally protected at the amino or carboxylic groups.

6. A process as claimed in claim 5, **characterized in that** a compound selected from the group consisting of 6-deoxy-6-iodo-β-cyclodextrin, epoxide 3A,2B-anhydrocyclodextrin and 6-amino-6-deoxy-β-cyclodextrin is used as functionalized β-cyclodextrin.

7. Pharmaceutical compositions containing one or more compounds of formula (I).

8. Pharmaceutical compositions as claimed in claim 7 with antioxidant (radical scavenger) activity.

9. Pharmaceutical compositions as claimed in claim 7 with anticataract activity.

## Patentansprüche

1. Verbindungen der Formel (I): 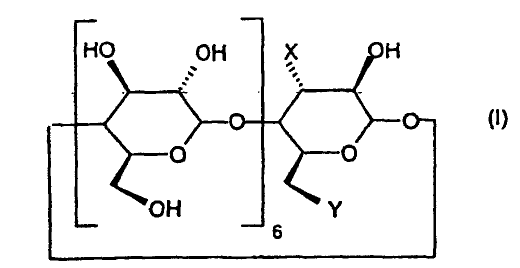 worin X und Y, die voneinander verschieden sind, eine Kette, die sich von Carnosin (β-Alanylhistidin) ableitet, oder eine OH-Gruppe sind.

2. Verbindung nach Anspruch 1, nämlich 3^{A}(R)-Desoxy-3^{A}(R)-(β-alanylhistidin)-2^{A}(S)-β-cyclodextrin der Formel (Ia)

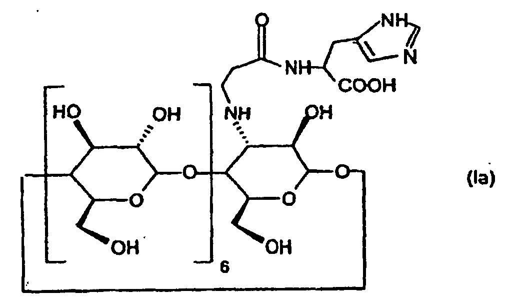

3. Verbindung nach Anspruch 1, nämlich 6-Desoxy-6-(β-alanylhistidin)-β-cyclodextrin der Formel (Ib)

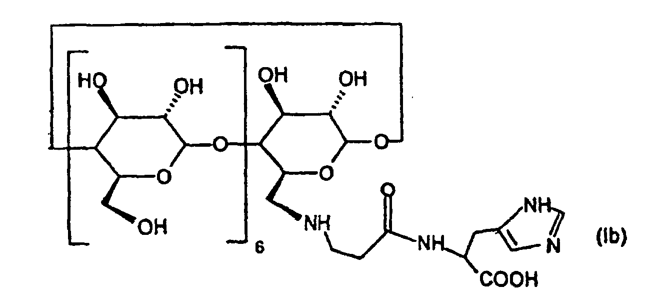

4. Verbindung nach Anspruch 1, nämlich 6-Desoxy-6-(β-alanylhistidylamino)-β-cyclodextrin der Formel (Ic)

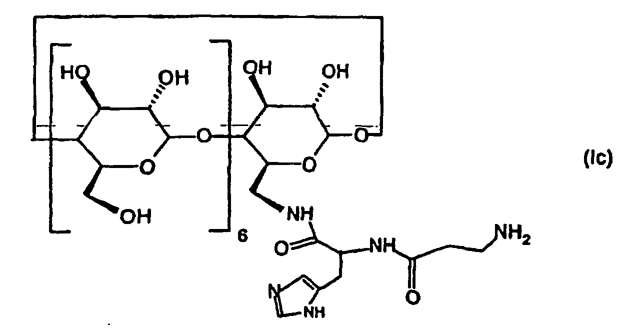

5. Verfahren zur Herstellung der Verbindungen nach Anspruch 1, **dadurch gekennzeichnet, dass** ein funktionalisiertes β-Cyclodextrin mit Alanylhistidin, das gegebenenfalls an den Amino- oder Carboxylgruppen geschützt ist, umgesetzt wird.

6. Verfahren nach Anspruch 5, **dadurch gekennzeich- net**, dass eine Verbindung, ausgewählt aus der Gruppe, bestehend aus 6-Desoxy-6-Iod-β-cyclodextrin, Epoxid-3A,2B-anhydrocyclodextrin und 6-Amino-6-desoxy-β-cyclodextrin, als funktionalisiertes β-Cyclodextrin verwendet wird.

7. Pharmazeutische Zusammensetzungen, die eine oder mehr Verbindungen der Formel (I) enthalten.

8. Pharmazeutische Zusammensetzungen nach Anspruch 7 mit Antioxidans-(Radikalfänger)Aktivität.

9. Pharmazeutische Zusammensetzungen nach Anspruch 7 mit Antikataraktaktivität.

## Revendications

1. Composés de formule (I) : 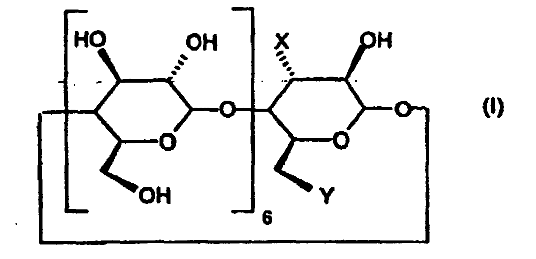 dans laquelle X et Y, qui sont différents l'un de l'autre, sont une chaîne provenant de carnosine (β-alanylhistidine), ou un groupe OH.

2. Composé selon la revendication 1, 3^{A}(R)-désoxy-3^{A}(R)-(β-alanylhistidine)-2^{A}(S)-β-cyclodextrine, de formule (la)

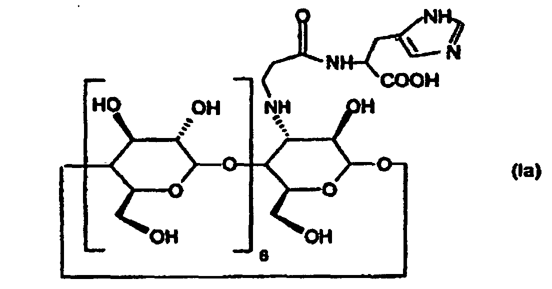

3. Composé selon la revendication 1, 6-désoxy-6-(β-alanylhistidine)-β-cyclodextrine, de formule (Ib)

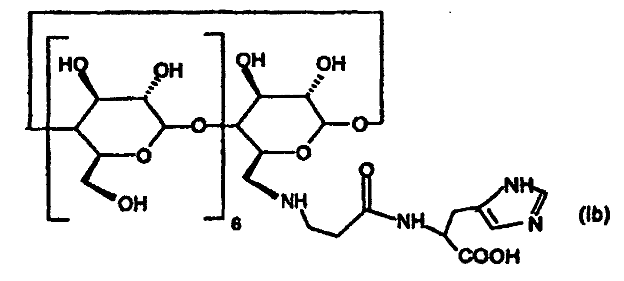

4. Composé selon la revendication 1, 6-désoxy-6-(β-alanylhistidylamino)-β-cyclodextrine, de formule (Ic)

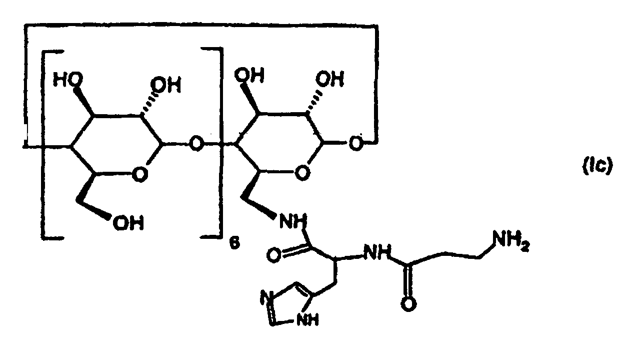

5. Procédé pour la préparation des composés selon la revendication 1, **caractérisé en ce qu'**une β-cyclodextrine fonctionnalisée est mise à réagir avec de l'alanylhistidine facultativement protégée aux groupes amino ou carboxyliques.

6. Procédé selon la revendication 5, **caractérisé en ce qu'**un composé choisi dans le groupe constitué par la 6-désoxy-6-iodo-β-cyclodextrine, la époxyde 3A,2B-anhydrocyclodextrine et 6-amino-6-désoxy-β-cyclodextrine est utilisé en tant que β-cyclodextrine fonctionnalisée.

7. Compositions pharmaceutiques contenant un ou plusieurs composés de formule (I).

8. Compositions pharmaceutiques selon la revendication 7, ayant une activité antioxydante (épuisant les radicaux).

9. Compositions pharmaceutiques selon la revendication 7, ayant une activité anticataracte.
